# EUROPEAN PATENT APPLICATION

(11) **EP 4 437 944 A1**
(43) Date of publication of application: **02.10.2024**
(21) Application number: 23166014.3
(22) Date of filing: 31.03.2023
(51) Int. Cl.: A61B 5/024, A61B 5/1455, A61B 5/00

(54) **SYSTEMS AND METHOD FOR DETERMINING THE QUALITY OF A PPG-SIGNAL**

(71) Applicant: Gabi SmartCare, 1435 Mont-Saint-Guibert (BE)
(72) Inventor: STAQUET, Olivier, 1410 Waterloo (BE)
(74) Representative: Calysta NV

(57) **Abstract**

A system for determining a signal quality of a PPG-signal, comprising: a receptor system configured to receive at least first radiation emitted by a first LED-source onto tissue of a patient, and configured to generate a first PPG-signal based on the first radiation, a control unit configured to: determine a time derivative function of the first PPG-signal; determine a skewness metric based on the time derivative function, wherein the skewness metric is a non-relative metric; convert the skewness metric into an SQI using a predetermined transfer function, wherein the SQI represents a quality of the first PPG-signal.

## Description

The present invention relates to the field of photoplethysmography (PPG) signals, for example used to determine one or more biometrics of a patient. The invention can e.g. be applied to a wearable device having a system to capture and/or emit a PPG-signal. The invention relates in particular determining the quality of a PPG-signal.

It is known that PPG-signals can be used to determine several biometrics of a patient, such as heart/pulse rate, respiratory rate, and oxygen saturation (SpO2). Radiation is usually emitted from one or more light emitting diodes (LEDs) onto tissue of the patient. The radiation reflected by the tissue or the radiation travelling through the tissue, is received by a receptor. The signal received by the receptor is generally referred to as the PPG-signal. Several characteristics of the PPG-signal depend on contents of the tissue, for example the blood in blood vessels. Based on this, the biometrics can be determined.

The quality of the PPG-signal can be affected by several factors and may be affected by noise. When the quality is not satisfactory, it may occur that the biometrics are determined incorrectly. Several methods and systems are known to determine the quality of the PPG-signal. However, these methods often require some time before they can produce a results, e.g. because an average of several periods of the PPG-signal is first determined to establish a basis to compare subsequent period with.

It is an object of the invention to overcome the disadvantages of the prior art, or at least provide an alternative to the prior art. It is in particular an object of the invention to enable a fast and accurate determination of the quality of the PPG-signal.

One or more objects of the invention are achieved with a system for determining a signal quality of a PPG-signal, comprising:
- a receptor system configured to receive at least first radiation emitted by a first LED-source onto tissue of a patient, and configured to generate a first PPG-signal based on the first radiation,
- a control unit configured to:
   - determine a time derivative function of the first PPG-signal;
   - determine a skewness metric based on the time derivative function, wherein the skewness metric preferably is a non-relative metric;
   - convert the skewness metric into an SQI using a predetermined transfer function, wherein the SQI represents a quality of the first PPG-signal.

The invention can thus relate to a system for determining a signal quality of a PPG-signal. When mentioned herein, PPG is used as acronym for photoplethysmography. The PPG-signal can e.g. be used for determining a biometric of a patient. The biometric may e.g. be a pulse rate, a respiratory rate, or a peripheral oxygen saturation (SpO₂) level. The system can e.g. be part of a wearable device, e.g. being configured to be worn on a limb, e.g. on an arm or leg. The system can e.g. be part of a watch, e.g. a smartwatch or sports watch. The patient can e.g. be a young child, e.g. a neonate or infant or child up to and including 5 years of age. A neonate is a new-born child, e.g. 28 days of age or younger. An infant is a young child, e.g. 2 years or age of younger.

First radiation is emitted by a first LED source. When mentioned herein, LED is used as acronym for Light Emitting Diode. An LED is configured to emit radiation when being subjected to an electric current. The radiation is electromagnetic radiation, generally light, which can be in the visible spectrum and/or in the non-visible spectrum. Although the term LED source is used in singular term, it is envisaged that in practice a single LED source may comprise a plurality of LEDs, e.g. being grouped and configured to emit radiation at the same wavelength. The first LED source is configured to emit the first radiation at a first wavelength. The first wavelength may e.g. be in the red-light spectrum, the infrared-light spectrum, the green-light spectrum, or the purple-light spectrum. Optionally, the first LED source is comprised by the system.

The first LED source is configured to emit the first radiation onto tissue of the patient. It is envisaged that the system is generally used on living patients, usually humans, in a non-invasive manner. The first radiation will therefore in those cases be emitted onto skin of the patient. The first radiation will (partially) penetrate into and through the tissue. The tissue may, besides the skin, also e.g. include membranes, muscles, blood vessels, blood, and tendons. A part of the first radiation is reflected by the tissue, a part is absorbed, and a part travels through the tissue.

The system comprises a receptor system. The receptor system can e.g. comprise one or more receptors, e.g. photoreceptors. The receptor system receives at least a part of the first radiation. Optionally, the receptor system is also configured to receive further radiation, e.g. second radiation at a second wavelength, e.g. from a second LED source. For example, in some embodiments, the system can comprise a first (photo)receptor configured to receive first radiation at a first wavelength, and a second (photo)receptor configured to receive second radiation at a second wavelength. The first wavelength can e.g. be different from the second wavelength. It is also possible that the system comprises one or more (e.g. broadband) (photo)receptors configured to receive radiation at least the first and second wavelength. Combinations of broadband (photo)receptor(s) and (photo)receptors for the first and/or second wavelength are also possible.

The receptor receives a part of the first radiation emitted by the first LED source. This can e.g. be the first radiation reflected by the tissue, or the first radiation travelling through the tissue. This may depend on the relative positioning of the receptor and the LED source. The receptor system generates a first PPG-signal based on the received first radiation. The receptor thus converts the received radiation into an (electrical) signal. The first PPG-signal is a so-called pleth, which can be used for photoplethysmography. The first PPG-signal is a photoplethysmography signal. Being emitted onto the tissue of the patient, the first PPG-signal depends on characteristics of said tissue. Blood flowing through blood vessels is a changing factor in the tissue. The changes caused by the changes of the blood (e.g. pressure, oxygen saturation, ...) can be used to derive biometrics (e.g. pulse rate, SpO2 level, ...) of the patient. It is noted that the system may comprise further (electronic) components (such as amplifiers) that are not mentioned herein, but known to the skilled person.

Whether or not the biometric is determined accurately, depends on the quality of the first PPG-signal. The quality of the first PPG-signal is affected by several factors, such as the signal to noise ratio and the pressure on the skin (e.g. the wearable device being arranged tighter or looser). It may, therefore, be advantageous to determine a signal quality of the first PPG-signal.

The system may comprise a control unit. The control unit can e.g. be configured to receive the first PPG-signal. The control unit is configured to determine a time derivative function of the first PPG-signal. The time derivative is the derivative of the first PPG-signal to time, in particular the first derivative. The time derivative function thus represents the (rate of) change of the first PPG-signal.

The control unit is further configured to a determine a skewness metric based on the time derivative function, wherein preferably the skewness metric is a non-relative metric. The skewness metric represents the skewness of the time derivative function, which is a measure of the asymmetry of the probability distribution of said time derivative function. Being a non-relative (or absolute) metric, the skewness metric is determined based on the time derivative function itself. Thus, the skewness metric is not determined by comparing with previously obtained time derivative functions or a general template. The skewness metric can e.g. be determined for a single period of the time derivative function (generally corresponding with a single period of the first PPG-signal).

The control unit is further configured to convert the skewness metric into an SQI. When mentioned herein, SQI is used as acronym for signal quality index. The SQI thus represents a quality of the first PPG-signal. The converting of the skewness metric to the SQI is done using a predetermined transfer function. For each value of the skewness metric, a predetermined value of the transfer function exits. By using the skewness metric as input parameter of the transfer function, the SQI is obtained. Being predetermined entails that the transfer function is not based on or derived from previous (periods of) first PPG-signals. The transfer function is thus fixed. That is, the transfer function is not changed during use, based on previous measurements or determinations. The transfer function can e.g. express the SQI as a meaningful number, e.g. within a range of 0-10, or 0-100, or as a percentage.

The determination of the SQI as explained is advantageous for several reasons. Firstly, the fact that a PPG-signal is skewed, is used to determine the SQI. Indeed, a PPG-signal is usually not normally distributed, and therefore thus skewed. The noise that reduces the quality of the signal on the other hand, can e.g. be white noise which is normally distributed and thus less skewed. Therefore, the skewness metric is affected by the noise. Using the skewness of the time derivative rather than the first PPG-signal has the advantage that the baseline is removed. By using the first derivative, the result is more robust to the variations, e.g. of the autonomic nervous system and to the artefacts of the signal. A further great advantage of the explained determination, is that the calculations and conversions are based on non-relative (or absolute) metrics and a predetermined transfer function. From the start of the system, an SQI can be determined. There is no need to first determine relative metrics/template or similar like in much of the prior art. The system can thus function faster.

The control unit can e.g. be configured to receive the first PPG-signal via a wired connection or via a wireless connection. For example, the control unit can be electrically connected to the receptor system, e.g. being arranged in a same housing. However, it is also possible that the control unit is arranged remotely. In such embodiments, the control unit can e.g. be arranged in a user equipment device arranged in the vicinity of the receptor system, or it is possible that the control unit is part of a cloud-based system.

The control unit may e.g. comprise a memory for storing (historic) data, data based on received PPG-signals, and/or executable instructions. The executable instructions may e.g. be non-transitory computer-readable instructions. The control unit may e.g. comprise a processing unit for processing the received PPG-signals and/or executable instructions, e.g. for determining the SQI, but also e.g. for obtaining a photoplethysmography signal, e.g. for determining one or more biometrics of the patient. The control unit may e.g. comprise a CPU. The control unit may e.g. comprise a RAM-memory. The control unit may e.g. comprise a flash-memory. The control unit may e.g. comprise or control a communication module, e.g. for communicating with an external device.

In embodiments, the transfer function is a non-unilinear function. Non- unilinear means that the transfer function is not a linear function with a single slope. Thus, the transfer function does not map every possible value of the skewness metric in the same way to an SQI. For example, a first range of possible skewness metric is mapped with a first conversion to an SQI, and a second range of possible skewness metric is mapped with a second conversion to an SQI. These embodiments that into account that the relation between the skewness metric and the quality of the first PPG-signal is not unilinear.

In embodiments, the transfer function comprises at least a first slope in a first skewness range, and a second slope in a second skewness range. The second slope is different from the first slope. Thus, the relation between the skewness metric and the SQI is different in the first skewness range when compared to the second skewness range. These embodiments that into account that the relation between the skewness metric and the quality of the first PPG-signal is not unilinear. In some embodiments, the transfer function may optionally comprise further slopes in further skewness ranges, e.g. a third slope in a third skewness range, e.g. a fourth slope in a fourth skewness range, etc.

In embodiments, the first skewness range is at smaller skewness than the second skewness range, and wherein first slope is steeper than the second slope. Thus, in the second skewness range the skewness metric is greater than in the first skewness range. However, in the first skewness range, the SQI increases faster than in the second skewness range. Optionally, the second skewness range is a narrower range than the first skewness range.

In embodiments, the transfer function comprises a maximal SQI at an optimal skewness, wherein the transfer function is positively sloped at a positive skewness range which is below the optimal skewness. Optionally, the transfer function is negatively sloped at a negative skewness range which is greater than the optimal skewness. Thus, the transfer function has a concave shape, wherein the SQI increases until the maximal SQI and then decreases again. It is noted that in the positive skewness range, it is possible that the slope is non-constant.

In embodiments, the transfer function is piece-wise linear function. A piece-wise linear function is a function with a plurality of linear parts, having different slopes.

In embodiments, the patient is a young child. For example, the system is configured to determine the signal quality of a PPG-signal based on first radiation emitted onto a young child. For example, the system is configured to be used when the patient is a young child. For example, the receptor system is configured to receive first radiation that is emitted onto tissue of a young child. For example, the first LED source is configured to emit the first radiation onto tissue of ta young child.

A young child can e.g. be a neonate or infant or child up to and including 5 years of age. A neonate is a new-born child, e.g. 28 days of age oryounger. An infant is a young child, e.g. 2 years or age of younger. The system may in particular be advantageous to determine the signal quality when the patient is a young child, as explained above.

The system for determining the signal quality may in particular be advantageous when the patient is a young child. The first PPG-signal will be different for a young child in comparison to adults. It is generally known that the PPG-signal is not completely sinusoidal. Normally there is a quick rise in the amplitude of the signal until a first peak, which corresponds with the systole. The amplitude of the signal then decreases (normally slower than the rising to the first peak), but not directly completely. Instead, a second, smaller rise, can usually be detected until. This rise goes towards a second peak, corresponding with the diastole. Then, the PPG-signal will decrease again.

For young children the second rise towards the second peak is much less pronounced. In some cases, a second rise may not even be visible. Instead the decrease is less steep. For young children, the PPG-signal thus has a different shape. In addition, the amplitude may also be different. Overall, the PPG-signal is less skewed for young children than for adults. Since a lower skewness is determined by physiological factors instead of mainly being determined by signal quality, the SQI can be adapted to this. The transfer function in the different embodiments explained accounts for this, e.g. by attributing a relatively higher SQI's to lower skewness metrics.

In embodiments, the control unit is configured to normalize the time derivative function before determining the skewness metric; and determine the skewness metric based on the normalized time derivative function. The normalization may e.g. include subtracting a mean value of the signal from the signal. The normalization may e.g. include scaling the signal to be within a predetermined range having a predetermined minimal value (e.g. -1 or -100) and a predetermined maximal value (e.g. +1 or + 100). The normalization makes it easier to compare results, e.g. between PPG-signals obtained at different wavelengths of wavelength, and/or on different moments, and/or different patients, and/or with different devices. The normalization makes the SQI that will later be determined easier to interpret, e.g. for a practitioner.

In embodiments, the control unit is configured to determine the SQI for a single period of the first PPG-signal. This advantageously allows to determine an SQI very quickly, e.g. after a single period of using the device. By determining a SQI for said single period, it e.g. possible to only use periods with a satisfactory quality for determining the biometric. Moreover, some biometrics are determined based on a single period (optionally for multiple PPG-signals at different wavelengths). A single period of the first PPG-signal usually corresponds to a single period of the time derivative function.

Generally a period of a signal is defined between the two occurrences of the signal. However, it is noted that the first PPG-signal will usually not be a perfectly periodic function, because subsequent periods are not equal. They can differ for several reasons, such as biometric reasons or noise. Also the time period of subsequent periods may differ, e.g. because the heart rate of the patient changes.

In embodiments, the control unit is further configured to determine at least one biometric of the patient based the first PPG-signal. Optionally, the control unit is further configured to determine a biometric quality based on the SQI. The biometric quality represents the quality with which the biometric can be determined. Since the SQI represents the quality of the first PPG-signal, it also affects the biometric quality. When the biometric is determined only based on the first PPG-signal, the biometric quality can e.g. be proportionally related to the SQI. For example, the biometric can be a pulse rate. When the SQI if low, this may indicate that the shape of the signal for that period was different than expected. This may e.g. imply that a beat in the pulse rate was missed, meaning that the corresponding determination of the pulse rate is of low quality and likely incorrect. Thus, the biometric quality will also be low.

In embodiments, the receptor is further configured to receive at least second radiation emitted by a second LED onto tissue of the patient, and configured to generate a second PPG-signal based on the second radiation. The control unit can e.g. be further configured to determine a time derivative function of the second PPG-signal. The control unit can e.g. be further configured to determine a second skewness metric based the time derivative function of the second PPG-signal, wherein the skewness metric is a non-relative metric. The control unit can e.g. be further configured to convert the second skewness metric into a second SQI using the predetermined transfer function, wherein the second SQI represents a quality of the second PPG-signal. The predetermined transfer function used for converting the second skewness metric into the second SQI may e.g. be the same transfer function that is used for converting the skewness metric into the SQI.

The second SQI can thus be determined similarly to the SQI of the first PPG-signal. It will be understood that any of the embodiments explained herein with respect to determining the SQI of the first PPG-signal can also be applied to determine the second SQI, mutatis mutandis. Similar advantages may be achieved with said embodiments.

In embodiments, the control unit is further configured to determine at least one biometric of the patient based on both the first PPG-signal and the second PPG-signal. Optionally, the control unit is further configured to determine a biometric quality based on both the SQI and the second SQI. For example, the biometric quality may be an average of the SQI and the second SQI. For example, the biometric quality may be a weighted average of the SQI and the second SQI, wherein e.g. the weighing is based on the method for determining the biometric. For example, the biometric can be a SpO2 level, which is often determined based on two PPG-signals generated by different wavelengths (e.g. the first PPG-signal being generated based on radiation in the red light spectrum and the second PPG-signal being generated based on radiation in the infrared light spectrum.

In embodiments, the first radiation is at a different wavelength than the second radiation. The first radiation can e.g. be at a first wavelength, and the second radiation at a second wavelength. The first wavelength may e.g. be in one of the red-light spectrum, the infrared-light spectrum, the purple-light spectrum, or the green-light spectrum, and second wavelength may e.g. be in another of the red-light spectrum, the infrared-light spectrum, the purple-light spectrum, or the green-light spectrum.

In embodiments, the system further comprises a first LED source configured to emit the first radiation at the first wavelength onto tissue of the patient. Optionally the system further comprises a second LED source configured to emit second radiation at a second wavelength onto the tissue of the patient. The first and/or second LED source may optionally be in accordance with any of the embodiments described herein and/or comprise any of the features mentioned herein with respect to LED sources.

It will be understood that although only first and second radiation have been referenced so far, similar principles can be applied for further radiation. For example, for third radiation at a third wavelength, e.g. emitted by a third LED source (optionally comprised by the system). The third wavelength may e.g. be in one of the red-light spectrum, the infrared-light spectrum, or the green-light spectrum For example, for fourth radiation at a fourth wavelength, e.g. emitted by a fourth LED source (optionally comprised by the system). Optionally, the fourth wavelength is in the same spectrum as one of the first, second, or third wavelength.

The invention may further relate to a wearable device for determining a biometric of a patient, comprising the system according to any of the embodiments described herein. Wearable means that the patient can wear the device on his body. The wearable device may e.g. comprise a housing, wherein the housing comprises the receptor system and the control unit. Optionally, the housing also comprises the first LED source (and optionally further LED sources when present). The wearable device may further comprise a band configured to be attached to the housing and to attach the housing to the tissue of the patient.

In embodiments, the wearable device is configured to be worn on an arm. The wearable device is e.g. configured to be arranged on an upper arm.

In embodiments, the wearable device is configured to be worn by e.g. by a young child, e.g. a neonate or infant or child up to and including 5 years of age. The system may in particular be advantageous to determine the signal quality when the patient is a young child, as explained above.

In embodiments, the wearable device comprises a communication module for communicating wirelessly with an external device. The external device may e.g. be user equipment device, such as a tablet, smartphone, or computer. The communication module may e.g. be configured to communicate using a short-range communication method, such as Bluetooth or Wi-Fi. The wearable device may e.g. be configured to communicate raw and/or processed data based the first PPG-signal via the communication module.

The invention further relates to a system for determining at least one biometric of a patient, comprising the wearable device according to one or more of the embodiments described herein, and a local user equipment device, wherein the user equipment device is configured to wireless communicate with the wearable device for receiving raw and/or processed data based the first PPG-signal. The local user equipment device may be configured to process raw/and or processed data, and/or to visualize the data received from the control unit. The local user equipment device may e.g. comprise a processing unit, memory, and/or screen. The local user device may e.g. be a smartphone or tablet.

In embodiments, the system further comprises a cloud-based infrastructure configured to receive raw and/or processed data from the local user equipment device, and comprising at least one server having a processing unit for further processing said raw and/or processed data. The cloud-based infrastructure may e.g. be configured to communicate with the local user equipment device via an internet communication, e.g. WI-FI, 3G, 4G, or 5G.

In embodiments, the system further comprises practitioner portal, configured to receive processed data from the cloud-based infrastructure, and configured to visualize data to a practitioner. Based on the visualized data, the practitioner can determine the health of the patient and propose the suitable medical treatment when required. The practitioner portal may e.g. be a web portal accessible via an internet browser, or an app portal accessible via an application or executable program on a user equipment device.

In embodiments, the system further comprising a caregiver portal, configured to receive processed data from the cloud-based infrastructure, and configured to visualize data to a caregiver. Based on the visualized data, the caregiver can access interesting information and e.g. determine whether a practitioner should be consulted. The caregiver portal may e.g. be a web portal accessible via an internet browser, or an app portal accessible via an application or executable program on a user equipment device.

The invention further relates to one or more methods. Although the method(s) can be performed with the system(s) or device(s) according to the invention; neither the system(s)/device(s), nor the method(s) are limited thereto. Features explained herein with reference to the system(s)/device(s) have the same meaning with respect to the method(s) unless explicitly defined otherwise. Features explained with reference to the system(s)/device(s) can be applied mutatis mutandis to the method(s) to achieve the similar advantages, and vice versa.

One or more objects of the invention can be achieved with a method for determining a signal quality of a PPG-signal, comprising the step of using a system according to any of the embodiments described herein.

One or more objects of the invention can be achieved with a method for determining a signal quality of a PPG-signal, wherein the method comprises the following steps:
- receiving at least first radiation emitted by a first LED onto tissue of a patient, and generating a first PPG-signal based on the first radiation,
- determining a time derivative function of the first PPG-signal;
- determining a skewness metric based the time derivative function, wherein the skewness metric is a non-relative metric;
- converting the skewness metric into an SQI using a predetermined transfer function, wherein the SQI represents a quality of the first PPG-signal.

In embodiments, the method includes using a system according to any of the embodiments described herein.

In embodiments, the transfer function is a non-unilinear function.

In embodiments, the transfer function comprises at least a first slope in a first skewness range, and a second slope in a second skewness range.

In embodiments, the first skewness range is at smaller skewness than the second skewness range, and wherein first slope is steeper than the second slope.

In embodiments, the transfer function comprises a maximal SQI at an optimal skewness, wherein the transfer function is positively sloped at a positive skewness range which is below the optimal skewness. Optionally, the transfer function is negatively sloped at a negative skewness range which is greater than the optimal skewness.

In embodiments, the transfer function is piece-wise linear function.

In embodiments, the method comprises a step of normalizing the time derivative function before determining the skewness metric, wherein the skewness metric is determined based on the normalized time derivative function.

In embodiments, the SQI is determined for a single period of the first PPG-signal.

In embodiments, the method comprises the following steps: receiving at least second radiation emitted by a first LED onto tissue of a patient, and generating a second PPG-signal based on the second radiation; determining a time derivative function of the second PPG-signal; determining a second skewness metric based the time derivative function, wherein the second skewness metric is a non-relative metric; converting the second skewness metric into a second SQI using the predetermined transfer function, wherein the second SQI represents a quality of the second PPG-signal.

In embodiments, the method further comprises the following steps: determining at least one biometric of the patient based on both the first PPG-signal and the second PPG-signal, and determining a biometric quality based on both the SQI and the second SQI.

In embodiments, the first radiation is at a different wavelength than the second radiation.

The invention further relates to non-transitory computer-readable instructions configured to, when executed, cause a control unit of a system for determining a signal quality of a PPG-signal according to any of the embodiments described herein, to perform one or more steps of a method according to any of the embodiments described herein. In particular, the computer-readable instructions may cause a control unit of the system to perform one or more steps and/or control one or more further components to perform one or more steps.

Exemplary embodiments of the invention are described using the figures. It is to be understood that these figures merely serve as example of how the invention can be implemented and are in no way intended to be construed as limiting for the scope of the invention and the claims. Like features are indicated by like reference numerals along the figures. In the figures:
Fig. 1a and fig. 1b: schematically illustrate a wearable device;
Fig. 1c: schematically illustrates a patient wearing the wearable device;
Fig. 2: schematically illustrates a system for determining a biometric;
Fig. 3a: schematically illustrates a method for determining an SQI and determining a biometric quality:
   Fig. 3b: schematically illustrates a method for determining a biometric quality for a biometric determined based on two PPG-signals;
Fig. 4a: schematically illustrates a PPG-signal obtained when the patient in a young child;
Fig. 4b: schematically illustrates a PPG-signal, a time derivative function of the PPG-signal, a skewness metric, and an SQI;
Fig. 4c: schematically illustrates a transfer function;
Fig. 5: schematically illustrates that the system can be used with a cloud-based infrastructure and a practitioner portal and/or a caregiver portal.

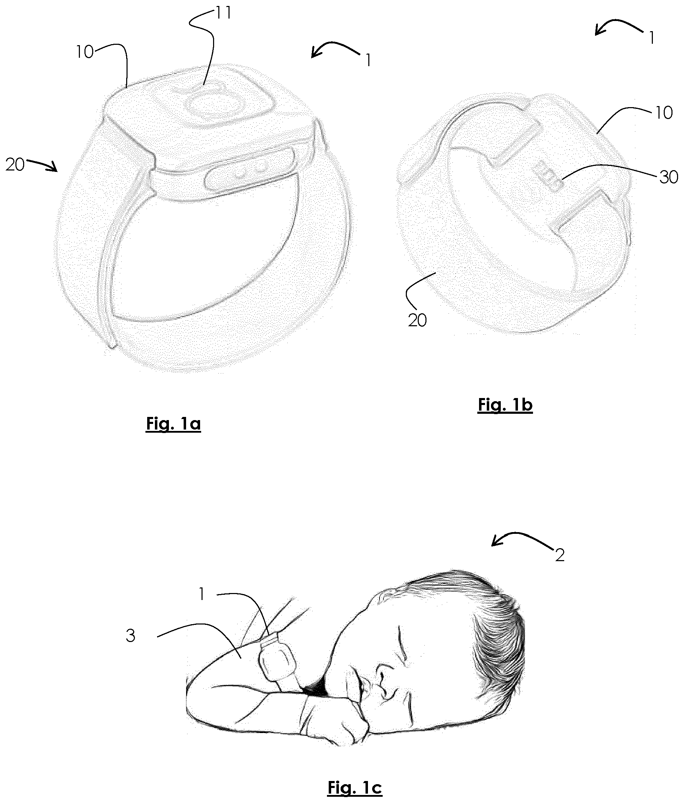

Fig. 1a and fig. 1b schematically illustrate a wearable device 1, and fig. 1c schematically illustrates a patient 2 wearing the wearable device 1. The wearable device 1 comprises a housing 10 and a band 20. The band 20 allows to attach the housing to the patient 2, in this case on the upper arm 3. The patient 2 in this case is a young child.

Fig. 1b illustrates that the wearable device has a PPG-system 30 on the bottom of the housing 10. The PPG-system 30 comprises a plurality of LED sources and photoreceptors. The photoreceptors receive radiation emitted by the LED sources and reflected by the tissue of the patient in the upper arm 3. Based on the reflected radiation, one or more biometrics can be determined.

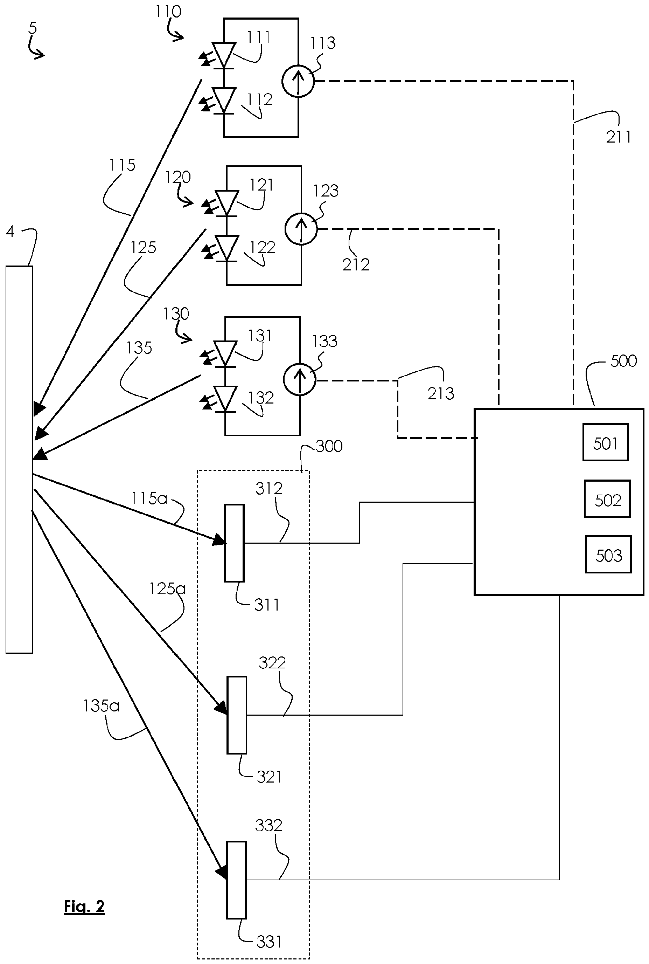

Fig. 2 schematically illustrates a system 5 for determining a biometric, that can e.g. be applied to the wearable device 1 shown in fig. 1a-1c. It is noted however, that the systems and methods explained in fig. 2 and generally in this text, can also be used in other devices, e.g. for older patients, and/or non-wearable devices, and/or for devices that analyse radiation that has travelled through tissue rather than being reflected by tissue.

The system 5 shown in fig. 2 comprises a first LED source 110, a second LED source 120, and a third LED source 130. The first LED source 110 is configured to emit first radiation 115 at a first wavelength, which can e.g. be in the red-light spectrum. The second LED source 120 is configured to emit second radiation 125 at a second wavelength, which can e.g. be in the infrared-light spectrum. The third LED source 130 is configured to emit third radiation 135 at a first wavelength, which can e.g. be in the green-light spectrum.

In the shown example, each of the LED sources 110, 120, 130 comprise two LEDs 111,112; 121,122; 131,132. It will be understood however, that this is only a schematical representation, and the exact number of LEDs per LED source 110, 120, 130 may differ and may depend on the device in which the system 5 is used. Moreover, it is possible that one of the LED sources 110, 120, 130 comprises a different number of LEDs than another of the LED sources 110, 120, 130.

The LEDs 111,112; 121,122; 131,132 will emit the respective radiation 115, 125, 135 when sufficient current flows through the respective LEDs 111, 112; 121,122; 131,132. Each LED source 110, 120, 130 comprises, therefore, a current source 113, 123, 133. The intensity of the emitted radiation 115, 125, 135 depends on the amount of current flowing through the respective LEDs 111,112; 121,122; 131,132.

The first, second, and third radiation 115, 125, 135 are emitted onto tissue 4 of the patient. The tissue 4 can e.g. be part of an upper arm, a lower arm, a wrist, a leg, a hand, a foot, a finger, an ear. The tissue 4 normally comprises at least the skin with which the radiation 115, 125, 135 comes into contact. The tissue 4 includes, besides the skin, also blood vessels with blood. The blood flowing through the blood vessel varies, e.g. in flow, pressure, and composition, depending on various biometrics of the patient. The variation of these aspects of the blood are represented in how much of the radiation 115, 125, 135 is reflected, absorbed, and completely travels through the tissue 4. Based on this, one or more biometrics of the patient can be determined. The tissue 4 may further also comprise e.g. include membranes, muscles, and tendons.

The first 115, second 125, and third radiation 135 are emitted sequentially. For example, first the first radiation 115 is emitted, thereafter the second radiation 125 is emitted, and finally the third radiation 135 is emitted. This is then repeated. When less or more LED sources 110, 120, 130 are present, this can be adapted accordingly.

Fig. 2 illustrates reflected first radiation 115a which is a part of the first radiation 115 that is reflected by the tissue 4. Similarly, reflected second radiation 125a and reflected third radiation 135a are illustrated. Although in the shown embodiment the reflected radiations 115a, 125a, 135a are used for determining the biometrics, the principles explained herein can also be applied to systems wherein radiations travelled through the tissue 4 is used for determining the biometrics.

A receptor system 300 receives a part of each of the first, second, and third radiation 115, 125, 135, wherein in this example said parts are the reflected first, second, and third radiation 115a, 125a, 135a. In the shown example the receptor system 300 comprises a first receptor 311 which receives the reflected first radiation 115a; a second receptor 321 which receives the reflected second radiation 125a; and a third receptor 331 which receives the reflected third radiation 135a.

It is noted that fig. 2 schematically illustrates each receptor 311, 321, 331 receiving one of the reflected first, second, and third radiation 115a, 125a, 135a. In practice, however, it is possible that the physical arrangement of the LED sources 110, 120, 130 and the receptor 311, 321, 331 entails that two or more (or each) of the receptors 311, 321, 331, receives two or more (or each) of the reflected radiations 115a, 125a, 135a. The first, second, and third receptor 311, 321, 331 can e.g. be photoreceptors configured to receive radiation at the respective wavelengths, and filter out radiation at other wavelengths.

In other embodiments, it is also possible that the receptor system 300 comprises one or more photoreceptors for receiving two or more of the reflected first, second, and third radiation 115a, 125a, 135a. In that case said photoreceptor can e.g. be a broadband photoreceptor.

The receptor system 300 is configured to generate a first PPG-signal 312 based on the reflected first radiation 115a. The receptor system 300 is configured to generate a second PPG-signal 322 based on the reflected second radiation 125a. The receptor system 300 is configured to generate a third PPG-signal 332 based on the reflected third radiation 135a. The respective PPG-signals 312, 322, 332 reflect how much of the respective reflected radiation 115a, 125a, 135a is received by the receptor system 300. The PPG-signals 312, 322, 332 thus comprise information that is related to one or more biometrics of the patient.

The system 5 further comprises a control unit 500. The control unit 500 receives the PPG-signals 312, 322, 332. The control unit 500 comprises a processing unit 501, e.g. for processing said PPG-signals 312, 322, 332, and determining one or more biometrics of the patient, e.g. a pulse rate, a SpO2 level, or a respiratory rate.. It is also possible, however, that the biometrics are determined by a further control unit (not shown). The further control unit may e.g. receive the PPG-signals 312, 322, 332 as raw data, or the control unit 500 may perform a preprocessing on the PPG-signals 312, 322, 333 and transmit processed data to the further control unit.

The control unit 500 comprises a memory 502. The memory 502 can e.g. store computer-readable instructions, based on which the control unit 500 can process the PPG-signals 312, 322, 332. The memory 502 can e.g. store raw data and/or processed data based on the PPG-signals 312, 322, 332. The control unit 500 comprises a wireless communication unit 503 which may e.g. be used for establishing a wireless connection. The wireless connection may use any suitable communication technique, e.g. including Wi-Fi, 3G, 4G, 5G, Bluetooth.

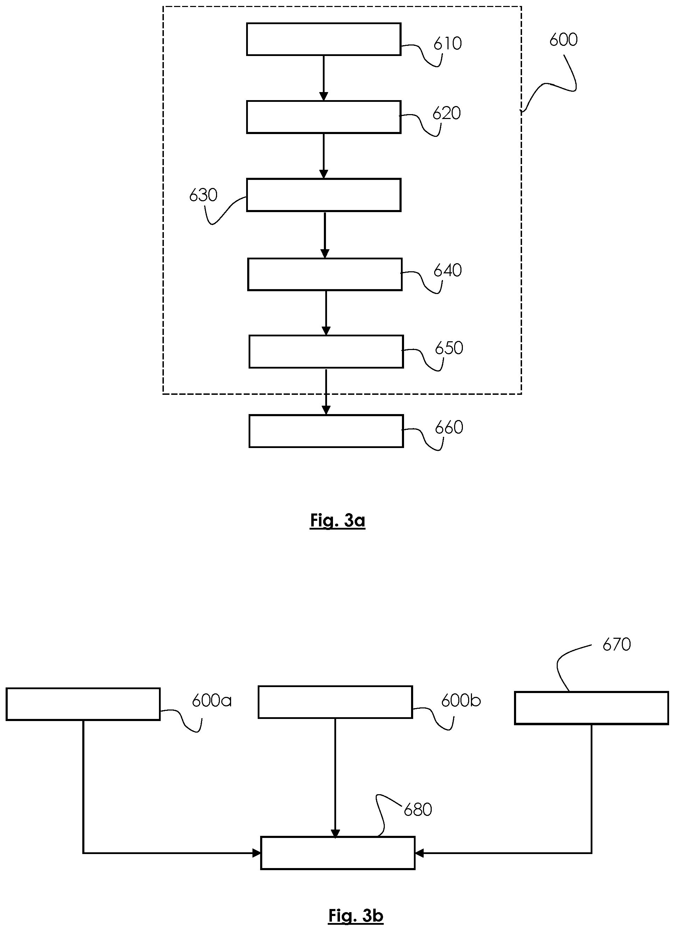

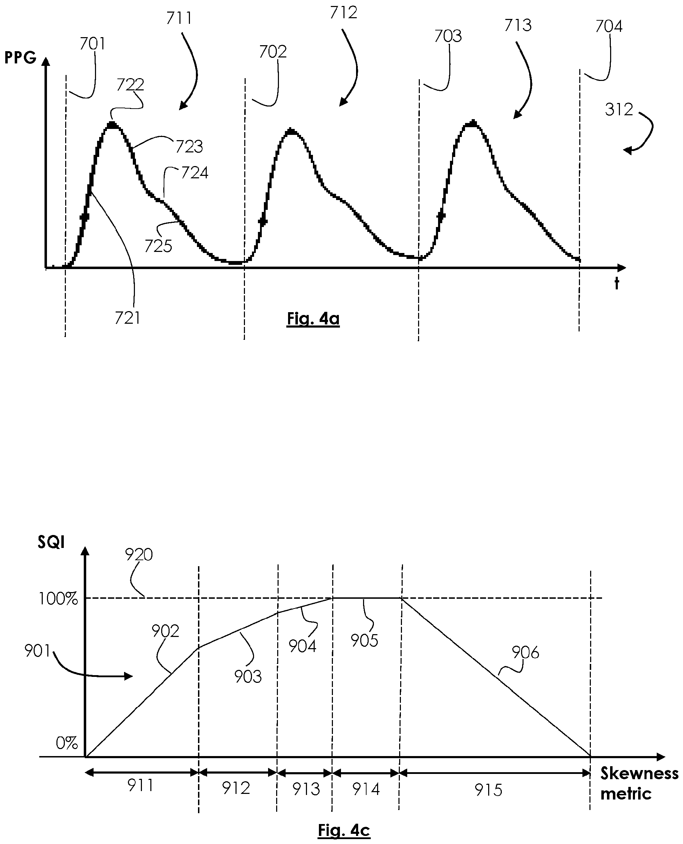

The control unit 500 can be configured to determine a signal quality for each PPG-signal 312, 322, 332. This will be explained with reference to figs. 2, 3a-3b, 4a-4c. Fig. 3a and fig. 3b illustrate methods that they can e.g. be performed with the system 5 shown in fig. 2. For example, the control unit 500 can be configured to perform one or more of the steps illustrated in fig. 3a-3b, or control other components to perform said steps. Non-transitory computer-readable instructions (which can e.g. be stored on memory 502) can e.g. configured to, when executed, cause the control unit 500 to perform such steps. Fig. 4a-4c show signals and functions that can be obtained or used to determine the quality of the PPG signal 312, 322, 332.

Since the signal quality is determined for an individual PPG-signal 312, 322, 332, it will be understood that these principles are not limited to the system 5 shown in fig. 2. Indeed, the signal quality can e.g. be determine in similar ways for system having less or more LED sources 110, 120, 130, or systems wherein the LED source(s) 110, 120, 130 are arranged in different physical housings, or systems wherein the radiation on which the PPG-signal 312, 322, 332 travelled through the tissue 4 rather than being reflected. The principles are below elaborated on with reference to the first PPG-signal 312, but it will be understood that they can be applied in similar ways to the second and/or third PPG signal 322, 332.

Fig. 3a illustrates a method 600 for determining a signal quality of a PPG-signal. In step 610, a receptor system 300 receives first radiation 115a. This can e.g. be reflected first radiation 115a received by the first photoreceptor 311 in fig. 2. Based on the received first radiation 155a, a PPG signal 312 is generated. Fig. 4a illustrates an example of how the first PPG-signal 312 can look when plotted in function of time. The horizontal axis represents the time, the vertical axis represents the amplitude of the PPG-signal.

In this example three periods 711, 712, 713 are plotted. The beginnings and ends of the periods are schematically indicated by dashed lines 701, 702, 703, 704. The periods 711, 712, 713 defined between two similar occurrences of the signal, in this case at the lowest amplitude. Although the term "period" is used herein, it is noted that the first PPG-signal 312 will usually not be a perfectly periodic function, because subsequent periods 711, 712, 713 are not completely equal to each other. They can differ for several reasons, such as biometric reasons or noise. Also the time period of subsequent periods 711, 712, 713 may differ, e.g. because the heart rate of the patient changes.

Indicated in the first period 711, is that each period the first PPG-signal 312 comprises a first rise 721 until a first peak 722. This corresponds with the systole of the heart, which affects the blood in the tissue, which in turn affects the first PPG-signal 312. The first rise 721 is a relatively quick rise. After the first peak 722, a decrease 723 in the amplitude of the first PPG-signal 312 can be identified. In the shown example, the patient is a young child. The inventors have found that this has an effect on the first PPG-signal 312, which is visible as a slower decrease 724. This corresponds with the diastole of the heart. In adults, the diastole is more pronounced and is usually visible as an increase in the amplitude of the PPG-signal. Finally a further decrease 725 in amplitude can be noted.

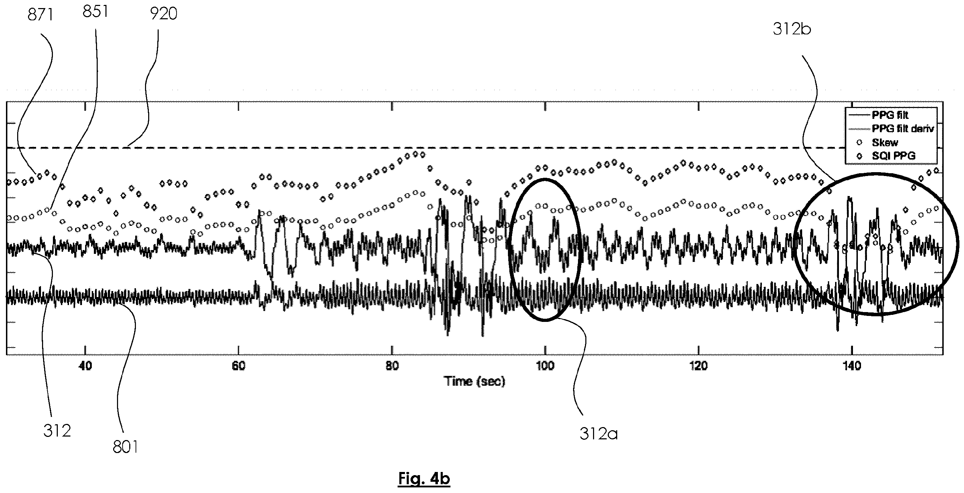

In practice, the PPG signal is not always as stable and well-pronounced as illustrates in fig. 4a. Fig. 4b illustrates an example of a first PPG-signal 312 measured in real life. For example, during the time periods indicated with 312a and 312b, the first PPG-signal 312 deviates quite strongly.

The horizontal axis in fig. 4b illustrates the time in seconds. The vertical axis reflects different scales for the different functions, because fig. 4b further illustrates a time derivative 801, a skewness metric, and an SQI. These functions will be elaborated on in more detail below.

In step 620 of fig. 3a, a time derivative function 801 of the first PPG-signal 312 is determined. The time derivative function 801 is shown in fig. 4b. Using the time derivative function 801 advantageously allows to remove the baseline. It can for example be seen in the region 312a that the first PPG-signal 312 is floating, which is removed in the first derivative function 801.

In step 630, the time derivative function 801 is normalized. Normalizing the time derivative function 801 allows to better compare the function and the quality determined in subsequent steps. It may e.g. be interesting to compare the quality between different PPG-signals 312, 322, 332 emitted by different LED sources 110, 120, 130.

Based on the normalized first PPG-signal, a skewness metric 851 is determined in step 640. The skewness metric 851 is plotted in fig. 4b, and represents the skewness of the time derivative function,. Said skewness is a measure of the asymmetry of the probability distribution of said time derivative function 801. The skewness can be determined with statistical methods that are well known. The skewness metric 851 is determined as a non-relative (or absolute) metric. Thus, the skewness metric 851 is not determined in comparison to a template, or an average based on previously obtained PPG-signals. This advantageously allows to quickly determine the quality, without the need to determine said average or template first.

The inventors have found that the skewness metric 851, being based on the first derivative function, is a good metric to determine the signal quality on. It can be seen in fig. 4a that the first PPG-signal 312 is not normally distributed. This is due to physiological aspects of the patient, and thus to be expected when the signal is of good quality. On the other hand, most of the noise that will reduce the quality of the first PPG-signal 312 is normally distributed. Thus, the lower the skewness metric 851, the lower the quality of the first PPG-signal 312. On the other hand, if the skewness metric is very large, this will usually also reflect a low quality of the first PPG-signal 312.

The skewness metric is converted into an SQI 871 in step 650. The SQI (acronym for Signal Quality Index) 871, represents the quality of the first PPG-signal 312. To convert the skewness metric 851 into the SQI 871, a transfer function 901 is used. Fig. 4c schematically illustrates an example of the transfer function 901. The transfer function 901 is predetermined and fixed, meaning that it will not change during use (e.g. based on previous measurements/calculations). For each value of the skewness metric 851, a predetermined value of the transfer function exits, defining the corresponding SQI. The SQI is expressed as a number that is easy for interpretation, in this case a percentage, with 100% being the best SQI possible.

In the example of fig. 4c, the transfer function 901 is a non-unilinear function. The transfer function 901 is a piece-wise linear function. In a first skewness range 911, the transfer function 901 comprises a first slope 902. In a second skewness range 912, the transfer function 901 comprises a second slope 903. The first skewness range 911 is at smaller skewness than the second skewness range 912. The first slope 902 is steeper than the second slope 903. A third skewness range 913 has an even smaller slope 904, reaching a fourth skewness range 914 having optimal skewness, and an SQI of 100% (represented by line 920 in fig. 4b and fig. 4c). While the first-third skewness ranges 911-913 have a positive slope, a fifth skewness range 915 has a negative slope 906. The fifth skewness range 915 is at skewness greater than the optimal skewness range 914.

The transfer function 901 in this embodiment is particularly advantageous when the patient is a young child. As illustrated with reference to fig. 4a, the first PPG-signal 312 obtained when the patient is a young child is different compared to adults. This results in a different skewness. A unilinear relation between the skewness metric and the SQI would, therefore, attribute incorrect quality to the skewness metric. This is resolved with the shown transfer function 901. This transfer function also takes into account that at higher skewness (see range 915), the quality decreases again. Furthermore, for negative skewness (below 0), the SQI is 0. That the correlation between the skewness metric 851 and the SQI is not unilinear can also be seen in region 312b in fig. 4b.

The skewness metric is determined for a single period 711, 712, 713. The SQI is consequently also determined for a single period 711, 712, 713. This may be advantageous, because some biometrics (e.g. pulse rate) of the patient can also be determined on a single period. A biometric quality can then be determined in step 660, based on the SQI. The biometric quality represents the quality of the determination of the biometric, based on the respective period 711, 712, 713 of the first PPG-signal.

Some biometrics (e.g. SpO2) are determined based on more than one PPG-signal 312, 322, 332. This can e.g. be done in step 670 shown in fig. 3b, where a biometric is determined based on the first PPG-signal 312 and the second PPG-signal. An SQI can then be determined for each PPG-signal. For example, a first SQI can be determined for the first PPG-signal 312 in step(s) 600a, which may include the steps shown in fig. 3a. A second SQI can be determined for the second PPG-signal 322 in step(s) 600b, which may include the steps similar to those shown in fig. 3a. In step 680 in fig. 3b, a biometric quality is determined for the biometric determined in step 670. The biometric quality is based on the first and second SQI, e.g. as a weighted average based on the contribution of the respective PPG-signal 312, 322 to the determination of the biometric.

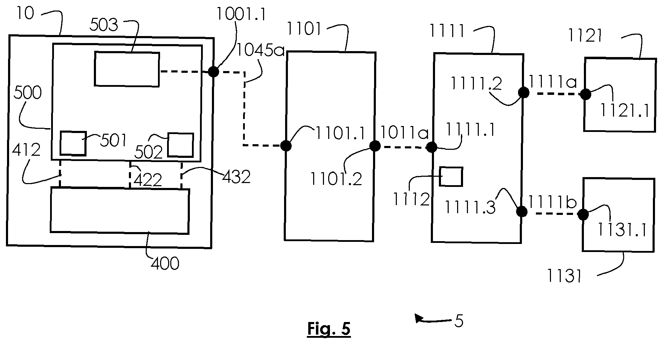

Fig. 5 schematically illustrates how the system 5 can be used with a cloud-based infrastructure 1111. A local user equipment device 1101 is illustrated. The local user equipment device 1101 can e.g. be a tablet or smartphone, and is arranged in the vicinity of the wearable device. Optionally the local user equipment device 1101 is pre-programmed by the operator of the system 5. The local user equipment device 1101 is configured to receive raw/and or processed data via communication signal 1045a from the control unit 500, e.g. via communication terminals 1001.1; 1101.1. The local user equipment device 1101 may be configured to process raw/and or processed data, and/or to visualize the data received from the control unit 500. The local user equipment device 1101 may e.g. comprise a processing unit, memory, and/or screen.

The system 5 further comprises a cloud-based infrastructure 1111 configured to receive a cloud communication signal 1101a from the local user equipment device 1101, e.g. via communication terminals 1101.2, 1111.1. The cloud communication signal 1101a may e.g. comprise raw and/or processed data. The cloud-based infrastructure 1111 comprises a server having a processing unit 1112 for further processing said raw and/or processed data. The cloud-based infrastructure 1111 may e.g. be configured to communicate the cloud communication signal 1101a with the local user equipment device 1101a via an internet communication, e.g. WI-FI, 3G, 4G, or 5G.

The system 5 further comprises a practitioner portal 1121, configured to receive processed data from the cloud-based infrastructure 1111. This is achieved by means of a practitioner communication signal 1111a communicated via communication terminals 1111.2, 1121.1. The practitioner portal 1121 is configured to visualize data to a practitioner. Based on the visualized data, the practitioner can determine the health of the patient and propose the suitable medical treatment when required. The practitioner portal 1121 may e.g. be a web portal accessible via an internet browser, or an app portal accessible via an application or executable program on a user equipment device.

The system 5 further comprising a caregiver portal 1131, configured to receive processed data from the cloud-based infrastructure 1111. This is achieved by means of a caregiver communication signal 1111b communicated via communication terminals 1111.3, 1131.1. The caregiver portal 1131 is configured to visualize data to a caregiver. Based on the visualized data, the caregiver can access interesting information and e.g. determine whether a practitioner should be consulted. The caregiver portal may e.g. be a web portal accessible via an internet browser, or an app portal accessible via an application or executable program on a user equipment device.

As required, detailed embodiments of the present invention are described herein; however, it is to be understood that the disclosed embodiments are merely examples of the invention, which may be embodied in various ways. Therefore, specific structural and functional details disclosed herein are not to be construed as limiting, but merely as a basis for the claims and as a representative basis for teaching those skilled in the art to practice the present invention in various ways in virtually any suitable detailed structure. Not all of the objectives described need be achieved with particular embodiments.

Furthermore, the terms and expressions used herein are not intended to limit the invention, but to provide an understandable description of the invention. The words "a", "an", or "one" used herein mean one or more than one, unless otherwise indicated. The terms "a multiple of", "a plurality" or "several" mean two or more than two. The words "comprise", "include", "contain" and "have" have an open meaning and do not exclude the presence of additional elements. Reference numerals in the claims should not be construed as limiting the invention.

The mere fact that certain technical features are described in different dependent claims still allows the possibility that a combination of these technical measures can be used advantageously.

A single processor or other unit can perform the functions of various components mentioned in the description and claims, e.g. of processing units or control units, or the functionality of a single processing unit or control unit described herein can in practice be distributed over multiple components, optionally physically separated of each other. Any communication between components can be wired or wireless by known methods.

The actions performed by the control unit can be implemented as a program, for example computer program, software application, or the like. The program can be executed using computer readable instructions. The program may include a subroutine, a function, a procedure, an object method, an object implementation, an executable application, a source code, an object code, a shared library / dynamic load library and / or other set of instructions designed for execution on a computer system.

A computer program or computer-readable instructions can be stored and / or distributed on a suitable medium, such as an optical storage medium or a solid-state medium supplied with or as part of other hardware, but can also be distributed in other forms, such as via internet or other wired or wireless telecommunication systems.

## Claims

1. A system for determining a signal quality of a PPG-signal, comprising:
• a receptor system configured to receive at least first radiation emitted by a first LED-source onto tissue of a patient, and configured to generate a first PPG-signal based on the first radiation,
• a control unit configured to:
• determine a time derivative function of the first PPG-signal;
• determine a skewness metric based on the time derivative function, wherein the skewness metric is a non-relative metric;
• convert the skewness metric into an SQI using a predetermined transfer function, wherein the SQI represents a quality of the first PPG-signal.

2. System according to claim 1, wherein the transfer function is a non-unilinear function.

3. System according to any of the preceding claims, wherein the transfer function comprises at least a first slope in a first skewness range, and a second slope in a second skewness range.

4. System according to the preceding claim, wherein the first skewness range is at smaller skewness than the second skewness range, and wherein first slope is steeper than the second slope.

5. System according to any of the preceding claims, wherein the transfer function is piece-wise linear function.

6. System according to any of the preceding claims, wherein the control unit is configured to normalize the time derivative function before determining the skewness metric, and determine the skewness metric based on the normalized time derivative function.

7. System according to any of the preceding claims, wherein the control unit is configured to determine the SQI for a single period of the first PPG-signal.

8. System according to any of the preceding claims, wherein
• the receptor is further configured to receive at least second radiation emitted by a second LED onto tissue of the patient, and configured to generate a second PPG-signal based on the second radiation,
• the control unit is configured to:
• determine a time derivative function of the second PPG-signal;
• determine a second skewness metric based the time derivative function of the second PPG-signal, wherein the skewness metric is a non-relative metric;
• convert the skewness metric into a second SQI using the predetermined transfer function, wherein the second SQI represents a quality of the second PPG-signal.

9. The system according to the preceding claim, wherein the control unit is further configured to
• determine at least one biometric of the patient based on both the first PPG-signal and the second PPG-signal, and
• determine a biometric quality based on both the SQI and the second SQI.

10. The system according to any of the preceding claims 8-9, wherein the first radiation is at a different wavelength than the second radiation.

11. The system according to any of the preceding claims, further comprising a first LED source configured to emit the first radiation at the first wavelength onto tissue of the patient; and optionally comprising a second LED source configured to emit second radiation at a second wavelength onto the tissue of the patient.

12. A wearable device for determining a biometric of a patient, comprising the system according to any of the preceding claims.

13. System for determining at least one biometric of a patient, comprising the wearable device according to one or more of the preceding claims, and a local user equipment device, wherein the user equipment device is configured to wireless communicate with the wearable device for receiving raw and/or processed data based on measurement of the at least one sensor of the wearable device.

14. Method for determining a signal quality of a PPG-signal, wherein the method comprises the following steps:
• receiving at least first radiation emitted by a first LED onto tissue of a patient, and generating a first PPG-signal based on the first radiation,
• determining a time derivative function of the first PPG-signal;
• determining a skewness metric based the time derivative function, wherein the skewness metric is a non-relative metric;
• converting the skewness metric into an SQI using a predetermined transfer function, wherein the SQI represents a quality of the first PPG-signal.

15. Non-transitory computer-readable instructions configured to, when executed, cause a control unit of a system according to any of the preceding claims 1-13 to perform a method according to claim 14.
